Europäisches Patentamt

(19) European Patent Office (11) Numéro de publication: **0 176 429**

Office européen des brevets **B1**

(12) **FASCICULE DE BREVET EUROPEEN**

(45) Date de publication du fascicule du brevet: **22.11.90** (51) Int. Cl.⁵: **A 61 K 39/395, A 61 K 39/44**

(21) Numéro de dépôt: **85401780.3**

(22) Date de dépôt: **13.09.85**

(60) Demande divisionnaire 89108697.7 déposée le **13/09/85.**

(54) **Compositions et procédé pour protéger les lymphocytes T contre l'agent étiologique des lymphadénopathies et du syndrome d'immunodépression acquise.**

(30) Priorité: **14.09.84 FR 8414172**

(43) Date de publication de la demande: **02.04.86 Bulletin 86/14**

(45) Mention de la délivrance du brevet: **22.11.90 Bulletin 90/47**

(84) Etats contractants désignés: **AT BE CH DE GB IT LI LU NL SE**

(56) Documents cités:
EP-A-0 018 794
EP-A-0 063 988

Science, vol. 220, 1983, pp.865-867.
Nature, vol. 335, 1988, pp.369-372.

(73) Titulaire: **INSTITUT PASTEUR 25-28, rue du Docteur Roux F-75724 Paris Cédex 15 (FR)**
(73) Titulaire: **CENTRE NATIONAL DE LA RECHERCHE SCIENTIFIQUE (CNRS) 15, Quai Anatole France F-75007 Paris (FR)**
(73) Titulaire: **UNIVERSITE PIERRE ET MARIE CURIE PARIS VI 4, place Jussieu F-75230 Paris Cedex 05 (FR)**

(72) Inventeur: **Klatzmann, David 84, quai de Jemmapes F-75010 Paris (FR)**
Inventeur: **Gluckman, Jean-Claude 70, Bld. du Port Royal F-75005 Paris (FR)**
Inventeur: **Montagnier, Luc 21, rue de Malabry F-92350 Le Plessis Robinson (FR)**

(74) Mandataire: **Gutmann, Ernest et al S.C. Ernest Gutmann - Yves Plasseraud 67, boulevard Haussmann F-75008 Paris (FR)**

## Description

L'invention est relative à un procédé et à des compositions pour protéger les lymphocytes T contre l'agent étiologique des lymphadénopathies et du syndrome d'immunodépression acquise.

L'agent étiologique des affections susdites a récemment été identifié. Il s'agit d'un virus qui a été dénommé LAV. Ce virus et divers variants de ce virus ont été complètement identifiés dans la demande de brevet Européen déposée le 14 septembre 1984 au nom de l'Institut Pasteur et sous le titre "Antigènes, moyens et méthode pour le diagnostic de lymphadénopathie et du syndrome d'immunodépression acquise", sous la priorité de la demande de brevet britannique 83 24800 déposée le 15 septembre 1983. On sait par ailleurs qu'un virus du même type a été isolé aux Etats-Unis d'Amérique. Il a été dénommé HTLV-III (SCIENCE, 1984, Vol. 224, *4648*, 497).

Pour la commodité du langage il sera fait référence dans ce qui suit au virus LAV, étant entendu que cette désignation s'étend en fait à tous les virus doués de propriétés équivalentes.

Il avait déjà été reconnu que les lymphocytes T-auxiliaires, plus particulièrement les cellules porteuses d'un déterminant OKT4⁺, ou "lymphocytes T4⁺", selon la désignation qui sera utilisée ci-après (cellules 3 Leu) semblaient former la principale cible du virus. Ces lymphocytes ont la particularité d'être reconnaissables par différents anticorps monoclonaux commercialisés par la firme ORTHO PHARMACEUTICAL CORPORATION sous la désignation OKT4. Ces anticorps monoclonaux ont été décrits plus particulièrement dans la demande de brevet européen 18794 déposée le 25 avril 1980. Il a également été établi que cet anticorps monoclonal reconnaissait plus particulièrement une protéine de poids moléculaire de l'ordre de 62 000 daltons, révélée après immunoprécipitation à partir d'un lysat de thymocytes humains marqués au borohydrure de sodium tritié, et ce après une oxydation douce en présence d'une faible concentration de périodate de sodium (C. TERHORST et coll. SCIENCE (1980), *209*, 520—521. Cette protéine a été dénommée "antigène T4" par les auteurs susdits. Elle sera ci-après encore désignée comme "protéine T4".

L'invention découle de la découverte que cette protéine T4 joue apparemment un rôle essentiel au niveau du processus d'infection des lymphocytes T par les susdits virus (ci-après plus généralement dénommés LAV). Plus particulièrement le processus d'infection des lymphocytes T paraît tributaire de l'interaction du virus avec la molécule T4, celle-ci paraissant jouer un rôle de récepteur pour le virus à la surface de la membrane. La reconnaissance de cette interaction découle de la constatation qui a été faite que l'infection d'une culture de cellules de lymphocytes T4⁺ par les virus LAV dans des conditions expérimentales qui normalement l'autorisent, ne se produit plus en présence d'anticorps monoclonaux anti-T4.

Il n'est en particulier plus possible de détecter l'immunofluorescence caractéristique de l'infection dans les conditions de culture décrites par E. L. Reinherz et Coll., Proc. Natl. Acad. Sci. USA (1979), Vol. 76, 4061—4065, dès que le milieu de culture contient également des anticorps monoclonaux anti-T4. Au contraire, des anticorps monoclonaux dirigés contre des antigènes autres que la protéine T4 présente sur les lymphocytes T ne perturbent en aucune façon l'infection dans les mêmes conditions des lymphocytes T par les virus LAV.

D'une façon générale l'invention concerne un procédé et des compositions dont le but est d'interférer avec le processeus infectieux, que ce soit au niveau de la pénétration des virus LAV dans les lymphocytes T ou de l'inhibition ou de la destruction du virus infectieux à l'intérieur même lymphocytes T infectés.

Le procédé selon l'invention pour la préparation d'une composition pharmaceutiqe pour maîtriser *in vivo* l'infection des lymphocytes T par les virus LAV, est caractérisé en ce que cette composition contient des anticorps anti-T4 ou un polypeptide possédant en commun avec l'anticorps anti-T4 un site de fixation sur la protéine T4, en association avec un véhicule pharmaceutiquement acceptable.

Cet anticorps anti-T4 est de préférence constitué par des anticorps monoclonaux présentant les caractéristiques essentielles de celui qui a été décrit dans la demande de brevet européen 18794, ou plus particulièrement encore par l'anticorps monoclonal qui est secrété par l'hybridome qui a été déposé par la société ORTHOPHARMACEUTICAL CORPORATION auprès de la collection dite "American Type Culture Collection" 12301 Parklawn Drive, ROCKVILLE, M D 20 852, aux Etats-Unis le 28 avril 1979. Cet hybridome est catalogué sous le No. ATCC CRL 8002.

Il va de soi que tout anticorps ayant des propriétés analogues peut être substitué à celui qui est secrété par le susdit hybridome. En particulier la composition selon l'invention peut contenir des anticorps polyclonaux formés au préalable contre des lymphocytes T4+, la seule condition étant naturellement que parmi ces anticorps polyclonaux il s'en trouvent certains qui puissent effectivement être fixes plus particulièrement sur la protéine T4. Toute autre substance susceptible de présenter une affinité semblable avec la protéine T4 peut être envisagée. En particulier, une substance adéquate pourrait être constituée par un polypeptide de plus petite taille que l'anticorps OK T4, ce polypeptide possédant, néanmoins en commun avec l'anticorps OK T4 le site de fixation sur la protéine T4.

Dans l'un de ses premiers modes de réalisation, l'invention concerne donc une composition pharmaceutique, de préférence injectable, constituée par l'association d'un anticorps anti-T4, tel qu'il a été défini ci-dessus, ou par une molécule douée de propriétés équivalentes, en association avec un excipient pharmaceutiquement acceptable (notamment d'une solution aqueuse, injectable, stérile, de préférence isotonique). Il va de soi que la composition doit être dosée de façon à assurer son efficacité au niveau de la prévention ou de l'inhibition *in vivo* de l'infection possible des lymphocytes T4 par le virus LAV. Plus particulièrement on observera que l'inhibition de l'infection supposera la réalisation d'une compétition efficace en faveur de la substance active vis-à-vis des virus, à l'égard desdits récepteurs.

La composition peut être utilisée soit seule, notamment lorsqu'elle est utilisée à titre préventif, soit en association ou en combinaison avec d'autres agents.

Par exemple l'invention concerne une composition associant des anticorps anti-T4 (ou des molécules équivalentes) et des lymphocytes provenant de donneurs sains. Les éléments de l'association peuvent être administrés simultanément ou d'une façon décalée dans le temps, pour aboutir aux résultats recherchés. Une telle composition sera d'un intérêt tout particulier dans les modes de traitement de patients se trouvant au dernier stade de la maladie, traitement qui implique des greffes de lymphocytes ou de moelle osseuse provenant d'un donneur sain. L'association à une telle greffe d'anticorps anti-T4 (ou substances analogues) par exemple au moment de la greffe peut avoir pour effet de protéger ces lymphocytes sains de l'infection à partir des lymphocytes infectés présents chez le malade. Les administrations peuvent également être décalées; en particulier des injections supplémentaires du principe actif de la composition selon l'invention après la greffe peuvent contribuer à l'inhibition stable de l'infection des lymphocytes greffés.

Il va de soi que les doses d'anticorps anti-T4 ou analogues, qu'il s'agisse de l'anticorps libre ou de l'anticorps couplé à des moyens permettant l'introduction d'un agent antiviral à l'intérieur des lymphocytes seront réglées par le clinicien en tenant compte de l'état du patient. On disposera d'un degré de liberté plus important, dans le cas où le patient doit ultérieurement recevoir une greffe de lymphocytes ou de moëlle osseuse provenant d'un individu sain.

L'invention peu également être mise en oeuvre *in vitro*, par exemple sur des prélèvements de lymphocytes contenant des cellules infectées obtenus à partir du patient, ces lymphocytes infectés étant alors mis en contact avec les compositions selon l'invention pendant le temps nécessaire à la destruction du virus ou des cellules infectées, avant d'être regreffés au patient.

Les anticorps monoclonaux du type sus-indiqué peuvent être cependant fixés sur une résine appropriée à la réalisation de chromatographies affines, le produit obtenu pouvant alors être utilisé, par exemple pour purifier le sang d'un patient, pour le débarrasser des cellules infectées, cette opération pouvant alors être réalisée dans le cadre d'une circulation extra-corporelle du sang.

L'invention permet de réaliser un procédé pour la préparation d'un système apte à permettre l'élimination du virus LAV contenue dans le plasma d'un patient atteint de lymphadénopathie ou du syndrome d'immunodépression acquise, ledit système comprenant des anticorps anti-T4 fixés sur une résine appropriée à la réalisation de chromatographie affine, permettant, lorsque l'on fait passer ce plasma au contact de ce système, de recueillir le plasma ainsi débarrassé de sa teneur en virus LAV.

Cette opération peut être réalisée par incorporation de la colonne d'affinité dans un circuit extra-corporel, le plasma à purifier étant prélevé sur le malade puis, après purification, réinjecté dans le malade.

On peut à l'inverse éliminer les lymphocytes infectés par passage du plasma du malade sur une colonne d'affinité portant des anticorps anti-T4.

Les différentes compositions évoquées ci-dessus peuvent être utilisées en tant que réactifs pour l'étude *in vitro* du comportement du virus LAV par rapport à des lymphocytes T4 dans des conditions déterminées.

## Revendications

1. Procédé pour la préparation d'une composition pharmaceutique pour maîtriser *in vivo* l'infection des lymphocytes T par les virus LAV, caractérisé en ce que ladite composition contient des anticorps anti-T4 ou un polypeptide possédant en commun avec l'anticorps anti-T4 un site de fixation sur la protéine T4, en association avec un véhicule pharmaceutiquement acceptable.

2. Procédé de traitement *in vitro* d'un prélèvement de lymphocytes T4 contenant des cellules infectées par des virus LAV, pour maîtriser l'infection des lymphocytes T par les virus LAV, caractérisé par la mise en contact de ces lymphocytes avec des anticorps anti-T4, pendant le temps nécessaire à la destruction des virus ou des cellules infectées.

## Patentansprüche

1. Verfahren zur Herstellung einer zum Beherrschen der LAV-Infektion der T-Lymphocyten pharmazeutischen Zusammensetzung, dadurch gekennzeichnet, dass die genannte Zusammensetzung anti-T4 Antikörper oder ein Polypeptid, das mit den anti-T4 Antikörpern eine gemeinsame Bindungstelle an dem T4-Protein aufweisst, in Verbindung mit einem pharmazeutisch verträglich Arzneimittelträger, enthält.

2. Verfahren zur *in-vitro* Behandlung einer T4-Lymphocyten Probe, die LAV-virus infizierten Zellen enthält, um die LAV-Infektion der T-Lymphocyten zu beherrschen, dadurch gekennzeichnet, dass die Lymphocyten während eine zur Zerstörung des Virus oder der infizierten Zellen ausreichende Zeitraum in Kontakt mit anti-T4 Antikörpern gebracht werden.

## Claims

1. Process for the preparation of a pharmaceutical composition for the *in vivo* control of infection of T-lymphocytes by the LAV-viruses, characterised in that the said composition contains anti-T4 antibodies or a peptide having, in common with the anti-T4 antibodies, a binding-site on the T4 protein, in association with a pharmaceutically aceptable vehicle.

2. Process for the *in-vitro* treatment of a sample of T4-lymphocytes containing cells infected by LAV-virus, for the control of infection of T-lymphocytes by the LAV-viruses, characterised by the contacting of these lymphocytes with anti-T4 antibodies for the time necessary to destroy the virus of the infected cells.